# EUROPÄISCHE PATENTSCHRIFT

(11) **EP 1 293 778 B1**
(45) Veröffentlichungstag und Bekanntmachung des Hinweises auf die Patenterteilung: **03.12.2008**
(21) Anmeldenummer: 02405733.3
(22) Anmeldetag: 29.08.2002
(51) Int. Cl.: G01N 33/18, G01N 27/49

(54) **Vorrichtung zur Konzentrationsüberwachung in Fluiden**
Apparatus for concentration monitoring in fluids
Appareil de contrôle de la concentration dans des fluides

(30) Priorität: 17.09.2001 CH 17052001
(43) Veröffentlichungstag der Anmeldung: 19.03.2003
(73) Patentinhaber: Baumer Holding AG, 8500 Frauenfeld (CH)
(72) Erfinder: Jobst, Gerhard, 79356 Eichstetten (DE); Moser, Isabella, 79356 Eichstetten (DE); Reetmeyer, Burkhard, 78462 Konstanz (DE)
(74) Vertreter: Frei, Alexandra Sarah

(56) Entgegenhaltungen:
- GB-A- 2 315 864
- US-A- 2 913 386
- E.A.H.HALL: "Biosensoren" 1995, SPRINGER - VERLAG , BERLIN HEIDELBERG NEW YORK , XP002373275 * Seite 108 - Seite 123 *

## Beschreibung

Die Erfindung betrifft eine Vorrichtung nach dem Oberbegriff des unabhängigen Patentanspruchs. Die Vorrichtung dient zur Überwachung der Konzentration flüchtiger organischer Verbindungen in Fluiden.

Die Konzentration von flüchtigen, organischen Verbindungen, zum Beispiel von Alkoholen in Fluiden, beispielsweise in wässerigen Lösungen wird je nach Anwendung mit verschiedenen Methoden, mit verschiedenen Genauigkeiten und mit verschiedenem Aufwand bestimmt und überwacht. Eine der einfachsten Methoden ist die Bestimmung des spezifischen Gewichtes der Lösung mittels Dichtespindel oder Aräometer, welche Methode aber keine zuverlässigen Werte ergibt, wenn die Alkoholkonzentration niedrig (<10%) ist und wenn die Lösung weitere Bestandteile (z.B. Salze) mit variierenden und unbekannten Konzentrationen beinhaltet. Ferner anwendbar sind optische Methoden, die im wesentlichen auf der Messung von Lichtabsroptionen bei spezifischen Wellenlängen beruhen. Diese Methoden verlangen im Falle von verschmutzten Lösungen eine sorgfältige Aufbereitung der Lösungsprobe und regelmässige Reinigung der Messanordnung. Ferner ist es bekannt, den alkoholischen Bestandteil durch eine semipermeable Wand oder durch aktives Austreiben mit Gas aus der wässerigen Lösung in einen Gasstrom zu übertragen und in diesem nach aus der Gasanalytik bekannten Analysemethoden zu analysieren, beispielsweise mittels Gaschromatographie oder elektrochemischen Methoden, wie beispielweise in der Publikation GB-A-2 315 864 beschrieben.

Ein Anwendungsgebiet der oben genannten Methoden ist die Überwachung und gegebenenfalls automatische Konstanthaltung (Regulierung auf einen Sollwert) der Konzentration von Isopropanol (2-Propanol) in sogenanntem Feuchtwasser, das in der Druckereiindustrie für die Behandlung von Druckwalzen für eine gleichmässige Benetzbarkeit eine wichtige Rolle spielt. Das Feuchtwasser weist neben einem Sollgehalt von Isopropanol von ca. 2 bis 8 Vol.% eine meist relativ hohe Verschmutzung insbesondere durch Farbreste auf, das heisst, es ist relativ trüb. Der Isopropanolgehalt des Feuchtwassers nimmt wegen der Flüchtigkeit des Isopropanols dauernd ab, so dass das Feuchtwasser für eine garantiert gute Druckqualität entweder einen dauernd überhöhten Isopropanolgehalt haben muss oder der Isopropanolgehalt überwacht und entsprechend immer wieder Isopropanol zugegeben werden muss. Da Isopropanol in der Arbeitsatmosphäre ein Gesundheitsrisiko darstellt und ein kostenmässig nicht zu vernachlässigender Hilfsstoff der Druckereiindustrie ist, liegt es im Interesse dieser Industrie, die Isopropanolkonzentration so nahe wie möglich am notwendigen Minimalwert zu halten, was aber eine hohe Konstanz dieser Konzentration, also auch eine entsprechend genaue und im wesentlichen kontinuierliche Überwachung dieser Konzentration erfordert.

Gemäss dem Stande der Technik wird der Isopropanolgehalt von Feuchtwasser mittels Aräometer gemessen oder optisch, wie beispielsweise in der Publikation DE-4324141 beschrieben. Die Dichtemessung ist dabei, wie bereits weiter oben erwähnt wenig genau und die optische Messung apparativ aufwendig.

Die Erfindung stellt sich nun die Aufgabe, eine Vorrichtung zur Überwachung der Konzentration flüchtiger organischer Verbindungen, insbesondere niedermolekularer Alkohole (Methanol, Ethanol, Propanol, Isopropanol, Butanole) in Fluiden, insbesondere in wässerigen Lösungen zu schaffen, wobei die Vorrichtung gemäss Erfindung mit einem geringen apparativen Aufwand eine im wesentlichen automatische Konzentrationsüberwachung erlauben, Messungen auch bei kleinen Konzentrationen (<10 Vol.%) mit Genauigkeiten von 10% (relativ) oder weniger ermöglichen und insbesondere für die Überwachung und gegebenenfalls automatische Regulierung des Isopropanolgehaltes in Feuchtwasser oder Ethanolgehaltes in einer wässrigen Lösung anwendbar sein sollen.

Diese Aufgabe wird gelöst durch die Vorrichtung zur Überwachung der Konzentration flüchtiger, organischer Verbindungen in Fluiden, wie sie in den Patentansprüchen definiert sind.

Das in der Vorrichtung durchgeführte Verfahren basiert auf einer Oxidation der zu überwachenden Verbindung an einer entsprechend polarisierten Elektrode und beschränkt sich aus diesem Grunde auf Fluide elektrochemisch oxidierbarer Verbindungen, wie beispielsweise Lösungen von primären oder sekundären Alkoholen.

Nach dem genannten Verfahren wird das Fluid, beispielsweise die wässerige Lösung oder das Gasgemisch, in Kontakt gebracht mit der einen Seite einer geeigneten, semipermeablen Membran, die für die zu überwachende Verbindung permeabel und für Wasser bzw. entsprechende Gase dicht ist und auf deren anderer Seite sich eine Elektrolytlösung befindet. Die durch die semipermeable Wand in die Elektrolytverbindung diffundierende Verbindung trifft auf eine gegenüber dem Elektrolyten bzw. gegenüber einer Gegenelektrode oder einer Referenzelektrode unter einem vorgegebenen positiven Potential stehende Messelektrode und wird an dieser (gegebenenfalls durch die Elektrodenoberfläche katalysiert) oxidiert. Der aufgrund der Oxidation durch die Messelektrode fliessende Strom wird als Messsignal weiterverarbeitet.

Komponenten des Fluids, die wie die zu überwachende Verbindung durch die semipermeable Membran diffundieren können und bei der vorgegebenen elektrischen Spannung der Messelektrode gleicherweise oxidiert werden, können die Messung verfälschen. Liegen solche Komponenten im Fluid mit einer gleichbleibenden Konzentration vor, kann ihr Einfluss mittels Messresultaten von Eichmessungen ausgeschaltet werden.

Als Elektroden eignen sich insbesondere mit Edelmetall, beispielsweise Platin oder Platinverbindungen (Platin-Rhodium-Verbindungen), beschichtete Elektroden. Dies sind vorzugsweise platinierte Carbonelektroden oder Platinelektroden, welche durch galvanische Abscheidung in geeigneter Rauhigkeit hergestellt werden können. Das Potential der Messelektrode wird mit Hilfe einer Potentiostatschaltung konstant gehalten. Dabei wird als Referenzspannung vorteilhafterweise nicht die Spannung der Gegenelektrode verwendet sondern die Spannung einer Referenzelektrode (z.B. mit konstantem Strom gespeiste Elektrode mit Wasserstoffschutzschicht). Die Polarisation der Messelektrode wird also vorzugsweise gegenüber einer Referenzelektrode konstant gehalten und dies unabhängig von der Leitfähigkeit einer Elektrolytlösung und vorzugsweise auch unabhängig von der Polarisation der Gegenelektrode.

Damit die katalytische Wirkung der Messelektrode während des Betriebs gleich bleibt, wird die Messelektrode vorteilhafterweise in an sich bekannter Weise in einer Mehrfachpulspolarographie (z.B. Tripelpulspolarographie) betrieben. Dabei wird jeder Messphase eine Vorphase oder mehrere Vorphasen (z.B. Reinigungsphasen und Konditionierungsphasen) vorgeschaltet. Während der Messphase liegt an der Messelektrode das oben genannte, auf einem vorgegebenen Wert konstant gehaltene Messpotential an, bei dem die zu überwachende Verbindung oxidiert wird. In den Vorphasen wird die Messelektrode regeneriert. Beispielsweise werden in einer Reinigungsphase durch ein hohes positives Potential an der Messelektrode angelagerte Stoffe (Oxidationsprodukte) vollständig oxidiert und dadurch entfernt und wird in einer Konditionierungsphase die katalytisch wirksame Elektrodenoberfläche beispielsweise durch kathodische Reduktion regeneriert (von Oxidschicht befreit).

An der Gegenelektrode läuft eine durch die Oxidation an der Messelektrode bedingte Reduktion ab. Damit auch diese möglichst vollständig abläuft und dadurch Folgemessungen unbeeinflusst bleiben, ist es vorteilhaft, der Gegenelektrode eine möglichst grosse Oberfläche zu geben.

In erfindungsgemässen Vorrichtung ist die Elektrolytlösung in einem geschlossenen Elektrolytraum enthalten und wird weder aktiv zirkuliert noch in irgend einer Art aufbereitet. In einem derartigen System ist es vorteilhaft, die Messelektrode nahe an der semipermeablen Membran anzuordnen und die Grösse der Membran derart an die Umsetzungskapazität der Messelektrode und deren Oberfläche anzupassen, dass der an der Messelektrode mögliche Umsatz verglichen mit der in die Elektrolytlösung diffundierenden Menge der zu überwachenden Verbindung gross ist. Auf diese Weise wird die ganze, in die Elektrolytlösung diffundierende Menge der Verbindung umgesetzt, so dass die Konzentration in der Elektrolytlösung im wesentlichen konstant auf Null bleibt und aufeinanderfolgende Messungen unabhängig voneinander bleiben (diffusionskontrollierte Messung). Um sicher zu stellen, dass dies mindestens in der unmittelbaren Umgebung der Messelektrode der Fall ist, kann ein Messbereich nahe an der semipermeablen Membran gegen Diffusion von zu überwachender Verbindung aus dem Rest der Elektrolytlösung durch eine gleich wie die Messelektrode polarisierte Schutzelektrode abgeschirmt werden.

Der Elektrolytraum ist in einen durch die semipermeable Membran und die Schutzelektrode begrenzten Messraum und einen Pufferraum aufgeteilt, wobei der Messraum verglichen mit dem Pufferraum sehr klein ist. Die Messelektrode ist im Messraum, die Gegenelektrode und die Referenzelektrode sind im Pufferraum angeordnet.

Der Messraum ist beispielsweise kanalförmig und Messelektrode und Schutzelektrode umspannen den Kanalinnenraum als Ringe. Die ebenfalls ringförmige Gegenelektrode umgibt die Mündung des kanalförmigen Messraumes in den Pufferraum. Die Gegenelektrode liegt vorteilhafterweise auf Masse. Die Referenzelektrode ist beispielsweise eine DHRE-Elektrode (dynamic hydrogen reference electrode), die dank Wasserstoffschutzschicht und Speisung aus einer Konstantstromquelle auf einem von der Leitfähigkeit der Elektrolytlösung unabhängigen, konstanten Potential liegt.

Das oben kurz beschriebene, diffusionskontrollierte Verfahren eignet sich für die Überwachung von sich relativ schnell ändernden Konzentrationen mittels kurz aufeinanderfolgender Messphasen. Dabei wird das sich während der Messphase einstellende Stromsignal oder eine daraus abgeleitete Messspannung als Messsignal weiter verwendet. Es ist auch möglich, die Messspannung über die Messphase zu integrieren. Für die Überwachung von sich langsamer verändernden Konzentrationen können die Messphasen auch zeitlich gespreizt werden. In solchen Fällen mag es vorteilhafter sein, die Messungen bei einem Diffusionsgleichgewicht zwischen Fluid und dem Elektrolytraum vorzunehmen und pro Messphase nur einen kleinen, das Gleichgewicht nur unwesentlich störenden Teil der zu überwachenden Verbindung im Elektrolytraum umzusetzen (mengenkontrollierte Messung). In diesem Falle ist keine Schutzelektrode vorzusehen und kann die Messelektrode irgendwo im Elektrolytraum angeordnet werden.

In der bevorzugten Ausführungsform der erfindungsgemässen Vorrichtung, die klein, kompakt und weitgehend mit bekannten Prozessen herstellbar ist, sind alle für die Sensorfunktion notwendigen Bestandteile auf einem Leiterplattensubstrat integriert. Das entsprechend beschichtete Substrat weist mindestens eine als Messraum dienende, durchgehende Öffnung (z.B. Bohrung) auf, an deren Innenoberfläche anliegend zwei ringförmige Elektroden (Mess- und Schutzelektrode) angeordnet sind. Auf der einen Substratseite ist die Bohrung von der semipermeablen Membran überspannt, auf der anderen Seite ist die Bohrung offen und die flächige Gegenelektrode erstreckt sich um die Mündung der Bohrung. Diejenige Substratseite, die die Gegenelektrode und gegebenenfalls auch die Referenzelektrode trägt, ist mit einer mit Elektrolytlösung gefüllten Blase überdeckt. Das Leiterplattensubstrat kann zusätzlich auch die für die Versorgung der Elektroden und für die Verarbeitung der Messsignale notwendigen elektrischen Schaltungen tragen.

Das wie oben beschrieben ausgerüstete Leiterplattensubstrat wird für die Konzentrationsüberwachung derart in das zu vermessende Fluid eingetaucht, dass mindestens die von der semipermeablen Membran überspannte Mündung der mindestens einen Bohrung mit Fluid bedeckt ist.

Es ist auch möglich, die erfindungsgemässe Vorrichtung so zu gestalten, dass die für die Sensorfunktion notwendigen Bestandteile, d. h. die Beschichtungen, nur auf einer Seite eines Leiterplattensubstrats angebracht sind. Eine als Messraum dienende Öffnung ist dann lediglich in den Beschichtungen angebracht, während das Substrat durchgehend gestaltet ist. Die eine Seite der Öffnung wird wiederum von einer semipermeablen Membran überspannt, während die andere Seite in ein Elektrolytreservoir mündet. Dieses wird dann im wesentlichen vom Leiterplattensubstrat begrenzt.

Während dem Betrieb kann sich die Zusammensetzung des Elektrolyten durch Reaktionsprodukte, die nicht durch die semipermeable Membran zurück ins Fluid diffundieren, verändern. Auch die Durchlässigkeit der semipermeablen Membran kann sich durch Verschmutzung verändern. Dadurch wird die Messung mit steigender Betriebsdauer unzuverlässiger. Die Einfachheit der Vorrichtung und ihrer Herstellung erlauben es aber, diese als Verbrauchsartikel zu betrachten und periodisch zu ersetzen.

Es zeigt sich, dass es mit einer Vorrichtung wie oben beschrieben mit einem Messraum in Form einer Bohrung durch ein beschichtetes Leiterplattensubstrat mit einem Durchmesser von ca. 0,3mm und mit einem Pufferraum in der Grössenordnung von 500 µl ohne weiteres möglich ist, während mindestens einem Monat im wesentlichen kontinuierlichen Betriebs Isopropanolkonzentrationen in Feuchtwasser zu messen mit einer absoluten Genauigkeit, die höher ist als 10% (relativ).

Eine bevorzugte Ausführungsform der erfindungsgemässen Vorrichtung wird im Zusammenhang mit den folgenden Figuren im Detail beschrieben. Diese Beschreibung bezieht sich auf die beispielhafte Anwendung der Vorrichtung gemäss Erfindung für die Überwachung des Isopropanolgehaltes von Feuchtwasser. Dies bedeutet in keiner Weise, dass die Erfindung nicht für andere, flüchtige organische Verbindungen, die elektrochemisch oxidierbar sind, z. B. elektrochemisch oxidierbare niedermolekulare wasserlösliche Verbindungen, anwendbar wäre. Die experimentell zu bestimmenden Parameter sind entsprechend anzupassen. Von den genannten Figuren zeigen:
- **Figur 1**: das Prinzip der erfindungsgemässen Vorrichtung;
- **Figuren 2 und 3**: einen Schnitt (Figur 2) durch und eine Draufsicht (Figur 3) auf eine bevorzugte Ausführungsform der erfindungsgemässen Vorrichtung, wobei nur der Bereich des Messraumes dargestellt ist;
- **Figur 4**: eine weitere, beispielhafte Ausführungsform der erfindungsgemässen Vorrichtung im Schnitt wie Figur 2;
- **Figur 5**: eine Potentiostatschaltung;
- **Figur 6a und 6b**: experimentell bestimmte Messkurven der erfindungsgemässen Vorrichtung gemäss Figur 2.

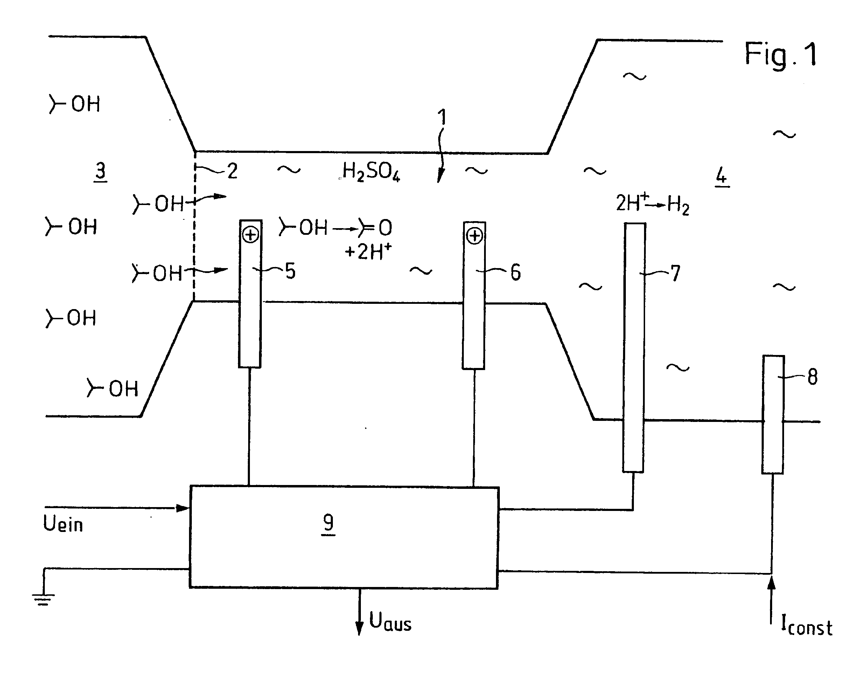

**Figur 1** zeigt schematisch das Prinzip die erfindungsgemässe Vorrichtung zur Überwachung des Isopropanolgehaltes in Feuchtwasser. Die Figur zeigt einen Messraum 1, der auf der einen Seite durch eine semipermeable Membran 2 gegen das Feuchtwasser 3 abgegrenzt ist und auf der gegenüberliegenden Seite gegen den Pufferraum 4 offen ist. Die semipermeable Membran 2 besteht für die vorliegende Anwendung beispielsweise aus Polydimethylsiloxan (PDMS). Messraum 1 und Pufferraum 4 sind mit einer geeigneten Elektrolytlösung, im vorliegenden Falle mit 5%iger Schwefelsäure gefüllt. Im Messraum 1 ist die Messelektrode 5 und die Schutzelektrode 6, die die Messelektrode 5 gegen den Pufferraum 4 abschirmt, angeordnet. An diesen beiden Elektroden liegt mindestens während den Messphasen ein vorgegebenes positives Potential an. Im Pufferraum 4 sind die auf Nullpotential liegende Gegenelektrode 7 und die mit einem konstanten Strom versorgte Referenzelektrode 8 angeordnet.

Die Elektroden sind über eine Potentiostat-Schaltung 9 miteinander verschaltet, wobei die Messelektrode 5 und die Schutzelektrode 6 parallel geschaltet sind. Die Aufgabe dieser Potentiostatschaltung 9 ist im wesentlichen die Konstanthaltung der Messelektrodenspannung unabhängig von der Leitfähigkeit der Elektrolytlösung auf einem vorgegebenen Wert und die Wandlung des Stromsignals in ein Spannungssignal Uₐᵤₛ. Dafür steht der Potentiostat-Schaltung eine Speisespannung Uₑᵢₙ zur Verfügung.

Isopropanolmoleküle diffundieren durch die semipermeable Membran 2 in den Messraum 1, wobei die pro Zeiteinheit in den Messraum 1 gelangende Menge an Isopropanol im wesentlichen proportional zum Konzentrationsgefälle über die semipermeable Membran 2 ist. An der Messelektrode 5 wird das Isopropanol beispielsweise zu Aceton oxidiert, wobei Protonen entstehen. Auch an der Schutzelektrode 6 wird Isopropanol zu Aceton oxidiert. Da die Messelektrode 5 derart ausgelegt und polarisiert ist, dass ihr Oxidationsvermögen grösser ist als die Menge des pro Zeiteinheit durch die Membran 2 diffundierenden Isopropanols, entspricht das Messsignal dieser Menge und somit der Konzentration ausserhalb der Membran 2. An der Schutzelektrode 6 wird lediglich gegebenenfalls aus dem Pufferraum 4 zurückdiffundierendes Isopropanol oxidiert.

An der Gegenelektrode 7 werden die durch die Oxidation an Mess- und Schutzelektrode entstandenen Protonen zu Wasserstoff reduziert.

Die Speisespannung Uₑᵢₙ wird während der Messphasen konstant gehalten und für andere Phasen der Mehrfachpulspolarographie entsprechend variiert. Für die Bestimmung einer geeigneten Polarisation der Messelektrode 5 wird bei bekannten Isopropanolkonzentrationen die Speisespannung kontinuierlich variiert (zyklische Voltametrie) und wird anhand der Messresultate ein Spannungsbereich ausgewählt, in dem das Messsignal möglichst proportional zur Isopropanolkonzentration ausserhalb der semipermeablen Membran 2 ist. Dasselbe gilt im Falle der Anwendung für andere Verbindungen als Isopropanol.

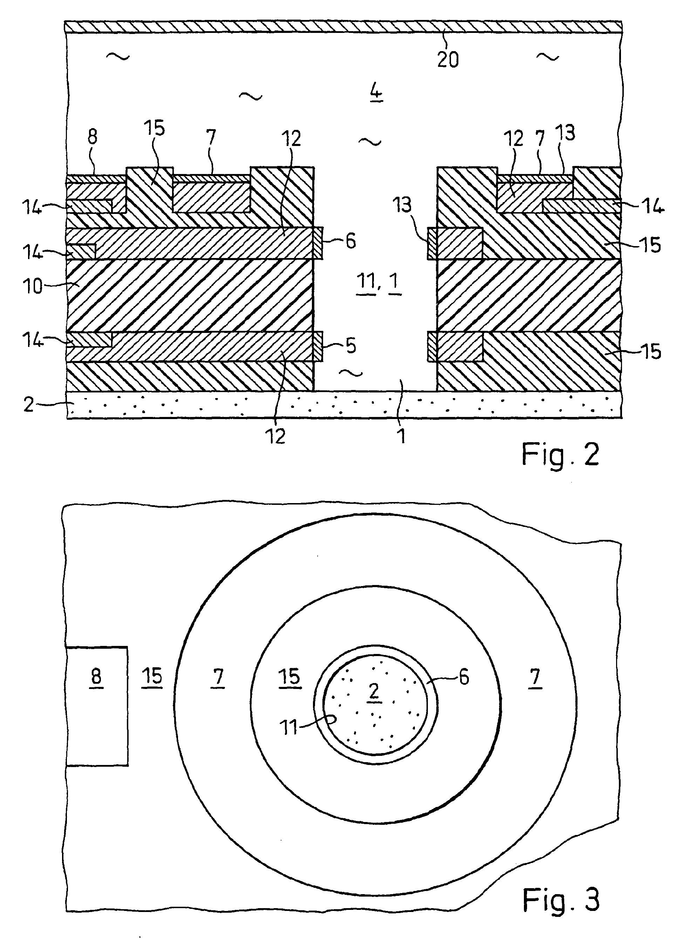

**Figuren 2 und 3** zeigen die bereits weiter oben kurz beschriebene, bevorzugte Ausrührungsform der erfindungsgemässen Vorrichtung, die im wesentlichen aus einem entsprechend beschichteten Leiterplattensubstrat 10 besteht. Dieses weist als Messraum 1 mindestens eine Bohrung 11 oder eine andere, geeignete durchgehende Öffnung auf. Von der Vorrichtung ist der Bereich einer derartigen Bohrung in der Figur 2 im Schnitt durch das Substrat 10 und in der Figur 3 als Draufsicht auf das Substrat dargestellt.

Im Schnitt der **Figur 2** sind das Leiterplattensubstrat 10 und die Bohrung 11 sichtbar. Unmittelbar auf dem Substrat 10 liegen entsprechend strukturierte Carbonschichten 12, die sich als Carbonelektroden mindestens rund um die Bohrung 11 erstrecken und an ihren Stirnseiten in der Bohrung 11 mit einer Edelmetallschicht versehen, typischerweise platiniert, sind. Diese Edelmetalloberflächen 13, z. B. Platinierungen, dienen als katalytisch wirkende Elektrodenoberflächen der Messelektrode 5 und der Schutzelektrode 6. Die Carbonschichten 12 sind mit Kupferleiterbahnen 14 kontaktiert. Die beiden Elektroden 5 und 6 und die entsprechenden Leiter sind abgedeckt mit Trockenresistschichten 15.

Auf der einen Seite ist auf der Trockenresistschicht 15 die semipermeable Membran 2 aufgezogen, die die Bohrung 11 überspannt und den Messraum 1 abschliesst.

Auf der anderen Seite sind auf bzw. in der Trockenresistschicht 15 die flächige und ebenfalls ringförmige Gegenelektrode 7 und die Referenzelektrode 8 positioniert, die ebenfalls als Carbonschichten 12 mit Edelmetalloberflächen 13, z. B. oberflächlichen Platinierungen, ausgebildet und mit einer Kupferleiterbahn 14 kontaktiert sind. Über Gegenelektrode 7, Referenzelektrode 8 und Bohrung 11 ist auf dieser Seite des Substrates 10 eine Blase oder ein Behälter 20 angeordnet, der mit Elektrolytlösung (z.B. 5% Schwefelsäure) gefüllt und geschlossen ist.

Die Ausbildung von zwei Lagen mit Carbonelektroden 12 auf der einen Seite des Substrates 10 erlaubt es, Gegenelektrode 7 und Referenzelektrode 8 derart auszugestalten, dass sie die Schutzelektrode 6 und deren Versorgungsleitungen überlappen. Nur so ist es möglich, die Gegenelektrode 7 als geschlossenen Ring auszubilden.

**Figur 3** zeigt eine Draufsicht auf das beschichtete Leiterplattensubstrat gemäss Figur 2 und zwar von der Seite des Pufferraumes 4. Durch die Bohrung 11 ist die semipermeable Membran 2 sichtbar. In der Bohrung 11 erstreckt sich die ringförmige Schutzelektrode 6, die die gleich ausgestaltete Messelektrode verdeckt. Um die Mündung der Bohrung 11 erstreckt sich die ringförmige Gegenelektrode 7 und daneben ist die Referenzelektrode 8 angeordnet.

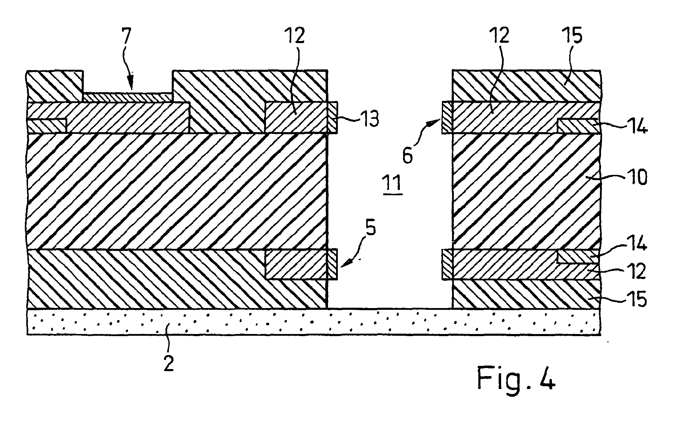

**Figur 4** zeigt in derselben Darstellungsweise wie Figur 2 eine weitere beispielhafte Ausführungsform der erfindungsgemässen Vorrichtung, die auf beiden Seiten des Leiterplattensubstrates 10 nur eine Elektrodenebene aufweist und aus diesem Grunde einfacher herstellbar ist. Gleiche Elemente sind mit gleichen Bezugsnummem bezeichnet wie in Figur 2.

Die Gegenelektrode 7 besteht in dieser Ausführungsform beispielsweise aus einer Mehrzahl von Ringsegmenten, wobei die Zuleitung zur Schutzelektrode 6 zwischen zwei derartigen Segmenten verläuft.

Zur Herstellung des in der Figur 4 dargestellten, beschichteten Leiterplattensubstrates wird ein allgemein gebräuchliches Leiterplattensubstrat 10 mit einer Dicke von beispielsweise 0,5mm verwendet. Auf diesem werden in an sich bekannter Weise die Kupfer- Carbon- und Trockenresistschichten 14, 12 und 15 aufgebracht und strukturiert. Dann wird die mindestens eine Bohrung 11 erstellt. Dann werden die freiliegenden Carbonoberflächen galvanisch platiniert oder mit einer anderen Edelmetallschicht versehen. Auf der einen Seite wird dann die semipermeable Membran 2 aufgezogen (z.B. durch Siebdruck) auf der anderen Seite der Behälter 20.

Für die Herstellung der Vorrichtung gemäss Figuren 2 und 3 sind die Schritte der Elektrodenherstellung für die Gegenelektrode 7 und die Referenzelektrode 6 auf der zweiten Elektrodenebene entsprechend zu repetieren.

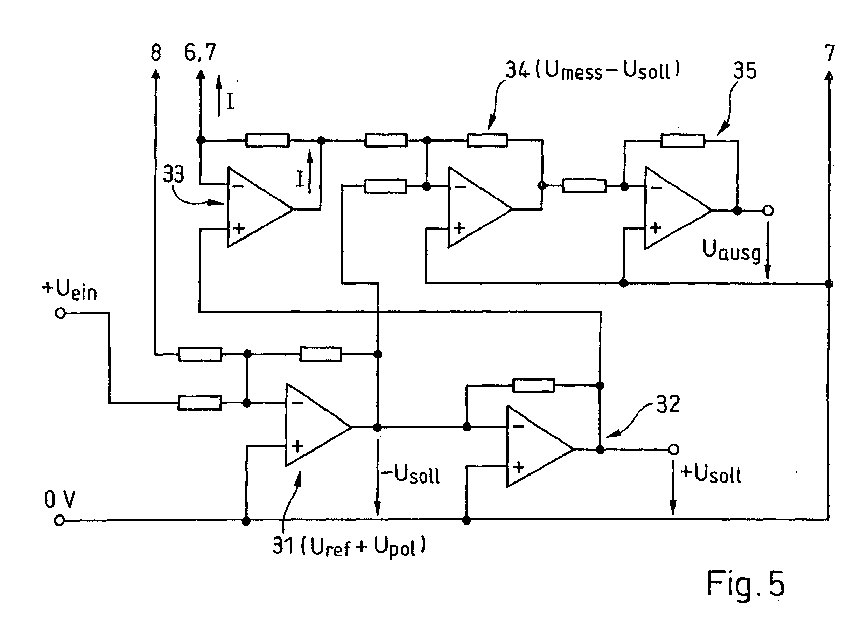

**Figur 5** zeigt eine beispielhafte Potentiostatschaltung, die für die erfindungsgemässe Vorrichtung abwendbar ist. Diese Schaltung weist im wesentlichen die folgenden Funktionen auf: einen Addierer 31, in dem eine Polarisationsspannung Uₚₒₗ zur Spannung U_{ref} der Referenzelektrode 8 zu Summe -Uₛₒₗₗ addiert wird, einen Sollwertermittler 32, der das Vorzeichen der Summe umkehrt, einen Spannungs/Strom-Wandler 33, der an die Messelektrode angeschlossen ist und ein Spannungssignal erzeugt, einen zweiten Addierer 34, mit dem aus dem Spannungssignal eine Messspannung Uₘₑₛₛ isoliert wird und eine Endstufe 35, an der das Messignal Uₐᵤₛ abgreifbar ist.

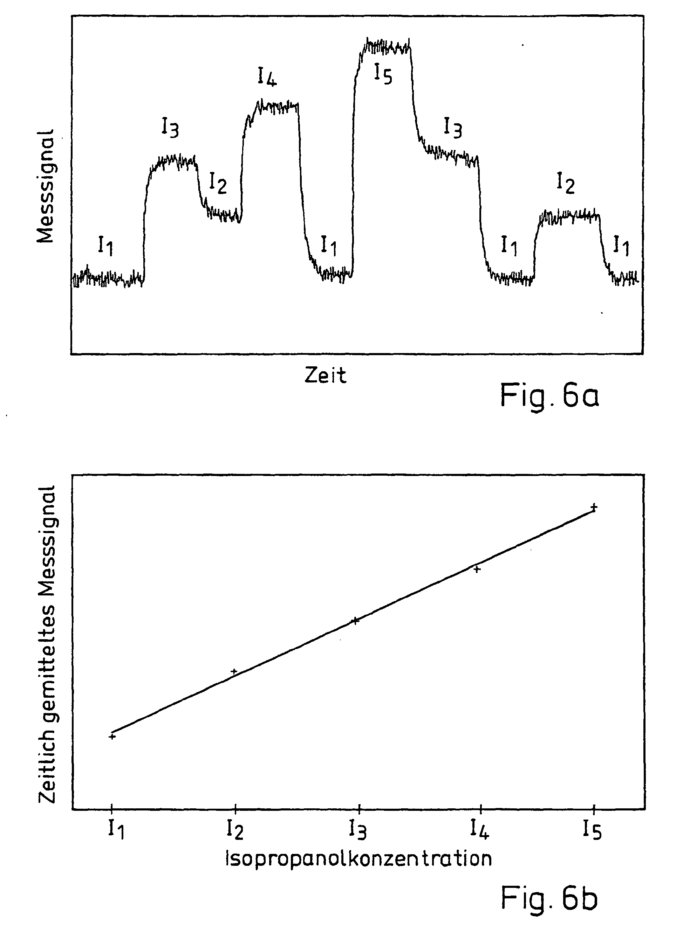

**Figur 6a und 6b** zeigen Messresultate, die mit der erfindungsgemässen Vorrichtung wie in Figur 2 gezeigt gemessen wurden. Es handelt sich dabei um einen Sensor, mit dem unterschiedliche Isopropanolkonzentrationen in einer wässrigen Lösung bestimmt wurden. Figur 6a zeigt das Messsignal in Abhängigkeit der Zeit, wobei die unterschiedlichen Signalintensitäten unterschiedlichen Isopropanolkonzentrationen I₁-I₅ zuzuordnen sind. Gut zu sehen ist die Reproduktionsfähigkeit der Signale: Gleiche Isopropanolkonzentrationen ergeben gleiche Messsignale, auch nach wiederholtem ändern der Isopropanolkonzentration von einem beispielsweisen Wert I₁ auf Werte I₂ - I₄ und wieder zurück auf den ursprünglichen Wert I₁.

In Figur 6b sind die zeitlich gemittelten Messsignale als Funktion der Isopropanolkonzentration I₁-I₅ in wässriger Lösung aufgetragen. Sehr deutlich ist die lineare Abhängigkeit des Messignals von der Konzentration zu sehen.

## Patentansprüche

1. Vorrichtung zur Überwachung der Konzentration einer flüchtigen organischen Verbindung in einem Fluid mittels elektrochemischer Umsetzung der Verbindung, welche Vorrichtung einen mit einer Elektrolytlösung gefüllten, geschlossenen Raum aufweist, in dem eine Messelektrode (5) und eine Gegenelektrode (7) angeordnet sind und der ein mit einer semipermeablen Membran (2) verschlossenes Fenster aufweist, derart, dass die Aussenseite der semipermeablen Membran (2) mit dem Fluid in Kontakt bringbar ist, und welche Vorrichtung ferner Mittel aufweist zur Erzeugung einer vorgegebenen Polarisation der Messelektrode (5) und zur Erzeugung eines Messsignals aus einem in der Messelektrode (5) fliessenden Strom, **dadurch gekennzeichnet, dass** der mit Elektrolytlösung gefüllte Raum aus einem kleineren Messraum (1) mit dem Fenster und der Messelektrode (5) und einem grösseren Pufferraum (4) mit der Gegenelektrode (7) besteht.

2. Vorrichtung nach Anspruch 1, **dadurch gekennzeichnet, dass** im Bereiche zwischen Messraum (1) und Pufferraum (4) eine gleich wie die Messelektrode (5) polarisierte Schutzelektrode (6) angeordnet ist.

3. Vorrichtung nach einem der Ansprüche 1 oder 2, **dadurch gekennzeichnet, dass** der Messraum (1) kanalförmig ist und dass die Messelektrode (5) und die Schutzelektrode (6) den Messraum (1) ringförmig umgreifen.

4. Vorrichtung nach Anspruch 3, **dadurch gekennzeichnet, dass** die Gegenelektrode (7) die Mündung des kanalförmigen Messraumes (1) in den Pufferraum (4) ringförmig umgibt.

5. Vorrichtung nach einem der Ansprüche 1 bis 4, **dadurch gekennzeichnet, dass** im Pufferraum (4) eine Referenzelektrode (8) angeordnet ist.

6. Vorrichtung nach Anspruch 5, **dadurch gekennzeichnet, dass** die Refeferenzelektrode (8) eine DRH (dynamit hydrogen reference) - Elektrode ist.

7. Vorrichtung nach einem der Ansprüche 1 bis 6, **dadurch gekennzeichnet, dass** alle Elektroden (5, 6, 7, 8) als galvanisch aufgebrachte Edelmetallschichten, bspw. platinierte Carbonelektroden oder Platinelektroden, ausgebildet sind.

8. Vorrichtung nach einem der Ansprüche 1 bis 7, **dadurch gekennzeichnet, dass** die Mittel zur Erzeugung einer vorgegebenen Polarisation der Messelektrode (5) und zur Erzeugung eines Messsignals aus einem in der Messelektrode (5) fliessenden Strom eine Potentiostat-Schaltung aufweisen.

9. Vorrichtung nach einem der Ansprüche 1 bis 8, **dadurch gekennzeichnet, dass** sie ein auf einer ersten und auf einer zweiten Seite beschichtetes Leiterplattensubstrat (10) aufweist mit einer als Messraum (1) ausgestalteten, durchgehenden Öffnung (11), die auf der ersten Seite durch die semipermeable Membran (2) überspannt ist, dass die Beschichtung auf der ersten und auf der zweiten Seite mindestens im Bereich der durchgehenden Öffnung als Messelektrode (5) und Schutzelektrode (6) dienende Carbonschichten (12), deren an die Öffnungen grenzende Oberfläche platiniert ist, aufweist, dass auf der zweiten Seite als Gegenelektrode (7) und als Referenzelektrode (8) dienende an ihrer äusseren Oberfläche ebenfalls platinierte Carbonschichtenbereiche (12) vorgesehen sind und dass auf der zweiten Seite ein mit Elektrolytlösung gefüllter Behälter (20) angeordnet ist, der die Mündung der durchgehenden Öffnung (11) und die als Gegenelektrode (7) und als Referenzelektrode (8) dienenden platinierten Carbonschichtenbereiche (12) überdeckt.

10. Vorrichtung nach einem der Ansprüche 1 bis 9, **dadurch gekennzeichnet, dass** die semipermeable Membran (2) aus Polydimethylsiloxan besteht.

11. Verwendung einer Vorrichtung nach einem der Ansprüche 1 bis 10 zur Überwachung der Konzentration von Isopropanol oder Ethanol in Feuchtwasser.

## Claims

1. Device for monitoring the concentration of a volatile organic compound in a fluid by means of electrochemical transformation of the compound, which device comprises a closed space filled with a electrolytic solution in which a measuring electrode (5) and a counter electrode (7) are arranged and which comprises a window closed with a semipermeable membrane (2), such that the outer side of the semipermeable membrane (2) may be brought into contact with the fluid and which device further comprises means for generating a predetermined polarization of the measuring electrode (5) and for generating a measuring signal from a current circulating in the measuring electrode, **characterized in that** the space filled with the electrolyte solution consists of a smaller measuring space (1) with the window and the measuring electrode (5) and a larger buffer space (4) with the counter electrode (7).

2. Device according to claim 1, **characterized in that** in the region between the measuring space (1) and the buffer space (4) a protecting electrode (6) polarized identically to the measuring electrode (5) is arranged.

3. Device according to one of claims I or 2, **characterized in that** the measuring space (1) has the shape of a channel and that the measuring electrode (5) and the protecting electrode (6) grip around the measuring space (1) in the manner of a ring.

4. Device according to claim 3, **characterized in that** the counter electrode (7) surrounds the mouth of the channel shaped measuring space (1) in the buffer space (4) in the manner of a ring.

5. Device according to one of claims 1 to 4, **characterized in that** in the buffer space (4) a reference electrode (8) is arranged.

6. Device according to claim 5, **characterized in that** the reference electrode (8) is a DRH (dynamic hydrogen reference) - electrode.

7. Device according to one of claims 1 to 6, **characterized in that** all electrodes (5, 6, 7, 8) are electro-plated noble metal layers, e.g. platinated carbon electrodes or platinum electrodes.

8. Device according to one of claims 1 to 7, **characterized in that** the means for generating a predetermined polarization of the measuring electrode (5) and for generating a measuring signal from a current circulating in the measuring electrode (5) comprise a potentiostat circuit.

9. Device according to one of claims 1 to 8, **characterized in that** it comprises a printed circuit board substrate (10), that is coated on a first and a second side and that comprises a continuous opening (11) designed as measuring space (1), which is covered by the semipermeable membrane (2), **in that** the coating on the first and on the second side comprises at least in the region of the continuous opening carbon layers (12) serving as measuring electrode (5) and protecting electrode (6), wherein the surface of the carbon layers adjoining the opening is platinated, **in that** on the second side carbon layer regions (12), that are again platinated on their outer surfaces, are provided to serve as counter electrode (7) and as reference electrode (8), and **in that** on the second side a container (20) filled with electrolyte solution is provided which covers the mouth of the continuous opening (11) and the platinated carbon layer regions (12) serving as counter electrode (7) and reference electrode (8).

10. Device according to one of claims 1 to 9, **characterized in that** the semipermeable membrane (2) consists of polydimethylsiloxan.

11. Use of a device according to one of claims 1 to 10 for monitoring the concentration of isopropanol or ethanol in dampening water.

## Revendications

1. Dispositif de surveillance de la concentration d'un composé organique volatil dans un fluide par conversion électrochimique du composé, lequel dispositif présente un espace fermé rempli d'une solution d'électrolyte, dans lequel une électrode de mesure (5) et une contre-électrode (7) sont disposées et qui présente une fenêtre fermée par une membrane semi-perméable (2) de telle sorte que le côté extérieur de la membrane semi-perméable (2) puisse être mis en contact avec le fluide, le dispositif présentant en outre des moyens qui établissent une polarisation prédéterminée sur l'électrode de mesure (5) et qui forment un signal de mesure à partir d'un courant qui s'écoule dans l'électrode de mesure (5),
**caractérisé en ce que**
l'espace rempli de solution d'électrolyte est constitué d'un petit espace de mesure (1) doté de la fenêtre et de l'électrode de mesure (5) et d'un grand espace tampon (4) qui présente la contre-électrode (7).

2. Dispositif selon la revendication 1, **caractérisé en ce qu'**une électrode de protection (6) polarisée de la même manière que l'électrode de mesure (5) est disposée dans la zone située entre l'espace de mesure (1) et l'espace tampon (4).

3. Dispositif selon l'une des revendications 1 ou 2, **caractérisé en ce que** l'espace de mesure (1) a la forme d'un canal et **en ce que** l'électrode de mesure (5) et l'électrode de protection (6) entourent l'espace de mesure (1) en anneau.

4. Dispositif selon la revendication 3, **caractérisé en ce que** la contre-électrode (7) entoure en anneau l'embouchure de l'espace de mesure (1) en canal dans l'espace tampon (4).

5. Dispositif selon l'une des revendications 1 à 4, **caractérisé en ce qu'**une électrode de référence (8) est disposée dans l'espace tampon (4).

6. Dispositif selon la revendication 5, **caractérisé en ce que** l'électrode de référence (8) est une électrode dynamique de référence à hydrogène ("dynamic hydrogen reference" - DRH).

7. Dispositif selon l'une des revendications 1 à 6, **caractérisé en ce que** toutes les électrodes (5, 6, 7, 8) sont formées de couches de métal précieux appliquées par électrolyse et sont par exemple des électrodes en carbone platiné ou des électrodes en platine.

8. Dispositif selon l'une des revendications 1 à 7, **caractérisé en ce que** les moyens de formation d'une polarisation prédéterminée d'une électrode de mesure (5) et de formation d'un signal de mesure à partir du courant qui s'écoule dans l'électrode de mesure (5) présentent un circuit de potentiostat.

9. Dispositif selon l'une des revendications 1 à 8, **caractérisé en ce que**
- il présente un support (10) en carte de circuit revêtu sur un premier et sur un deuxième côté et traversé par une ouverture (11) configurée comme espace de mesure (1) et recouverte sur le premier côté par la membre semi-perméable (2),
- **en ce que** le revêtement présente sur le premier et sur le deuxième côté, au moins dans la partie occupée par la perforation, des couches de carbone (12) qui servent d'électrode de mesure (5) et d'électrode de protection (6) et dont la surface adjacente à l'ouverture est platinée, - **en ce que** sur le deuxième côté sont prévues des parties (12) en couche de carbone également platiné sur leur surface extérieure et servant de contre-électrode (7) et d'électrode de référence (8) et
- **en ce que** sur le deuxième côté est disposé un récipient (20) rempli d'une solution d'électrolyte, et qui recouvre l'embouchure de la perforation (11) et les parties (12) en couche de carbone platinées qui servent de contre-électrode (7) et d'électrode de référence (8).

10. Dispositif selon l'une des revendications 1 à 9, **caractérisé en ce que** la membrane semi-perméable (2) est constituée de polydiméthylsiloxane.

11. Utilisation d'un dispositif selon l'une des revendications 1 à 10 pour la surveillance de la concentration d'isopropanol ou d'éthanol dans de l'eau de condensation.
